Europäisches Patentamt

European Patent Office

Office européen des brevets

(19)

(11) Veröffentlichungsnummer: **0 087 060**
**B1**

(12) # EUROPÄISCHE PATENTSCHRIFT

(45) Veröffentlichungstag der Patentschrift:
16.07.86

(51) Int. Cl.⁴: **A 61 K 7/021**

(21) Anmeldenummer: 83101210.9

(22) Anmeldetag: 09.02.83

(54) Fluoreszierende Schminke.

(30) Priorität: 10.02.82 DE 3204636

(43) Veröffentlichungstag der Anmeldung:
31.08.83 Patentblatt 83/35

(45) Bekanntmachung des Hinweises auf die Patenterteilung:
16.07.86 Patentblatt 86/29

(84) Benannte Vertragsstaaten:
**BE DE FR GB IT NL SE**

(56) Entgegenhaltungen:
**BE - A - 510 020**
**FR - A - 848 706**
**FR - A - 1 025 142**
**FR - A - 1 181 860**
**GB - A - 759 385**
**LU - A - 50 998**

**CHEMICAL ABSTRACTS, Band 96, Nr. 20, Mai 1982,**
**Seite 387, Nr. 168537m, Columbus, Ohio, USA**
**CHEMICAL ABSTRACTS, Band 80, Nr. 14, 8. April 1974,**
**Seite 238, Nr. 74268s, Columbus, Ohio, USA**

(73) Patentinhaber: **A.W. Faber - Castell GmbH & Co.,**
**Nürnberger Strasse 2, D-8504 Stein (DE)**

(72) Erfinder: **Jankewitz, Axel, Kuckucksweg 21,**
**D-8510 Fürth-Oberführberg (DE)**

## Beschreibung

Die Erfindung betrifft Schminken. Zu dieser Gruppe der Schönheitsmittel gehören alle festen, pastösen oder flüssigen Zubereitungen, die zur künstlichen Färbung der Körperoberfläche, also der Haut, der Nägel, der Lippen, aber auch der Wimpern dienen.

Die meisten Schminken enthalten ausser einer Schminkengrundlage noch weitere Zusätze, wie beispielsweise ätherische Öle oder sonstige Duftstoffe, pflegende Wirkstoffe u. dgl.

Die bekannten Schminken enthalten ausserdem Farbstoffe, welche ihre natürliche Eigenfarbe in unverfälschtem Zustand nur in Gegenwart von weissem Licht zeigen. Da weisses Licht insbesondere abends oder im Theater nicht zur Verfügung steht, sondern dort Licht mit stark erhöhtem Gelb- und Rotanteil verwendet wird, ist die Farbzusammensetzung vieler Schminken hierauf abgestimmt. Mit der Änderung der Lichtzusammensetzung ändert sich dann in bekannter Weise auch die Eigenfarbe der Schminke. Eine beträchtliche Farbänderung bei Bestrahlung, insbesondere eine solche, die nur die geschminkten Stellen des Körpes betrifft, ist aber auf diese Weise nicht zustande zu bringen.

Aus der FR-A 848 706 sind Schminken bekannt, welche bei Bestrahlung mit ultraviolettem Licht, sogenanntem Schwarzlicht, aufleuchten und dadurch ihre Eigenfarbe ändern. Die Schminken enthalten hierzu bestimmte fluoreszierende Stoffe, wie beispielsweise Auramin-Derivate, Eosine und Erythrosine, Rhodamine und ähnliches. Nachteilig bei diesen Zusammensetzungen ist jedoch der Umstand, dass die genannten Stoffe entweder nur mit geringer Leuchtkraft fluoreszieren oder eine Fluoreszenzfarbe zeigen, welche in Mischung mit anderen, bei Schminken üblichen Farbstoffen unterdrückt, stark verfälscht oder ausgelöscht wird.

Aus der FR-A 1 025 142 sind Mittel zur Schönheitspflege, beispielsweise Creme-Produkte u. dgl. bekannt, die ebenfalls Stoffe enthalten, welche unter bestimmten Umständen fluoreszieren. Im einzelnen sind dort insbesondere Stilben-Derivate genannt, aber auch Benzimidazol-Derivate, Thiazole und ähnliches. Abgesehen davon, dass diese Stoffe physiologisch bedenklich sind und in Hautpflegemitteln, Schminken u.dgl. nicht verwendet werden sollten, weisen aber auch diese Stoffe, insbesondere Stilben-Derivate, ungeeignete Fluoreszenzfarben auf.

In der FR-A 1 181 860 sind ebenfalls kosmetische Präparate mit den genannten fluoreszierenden Eigenschaften beschrieben. Auch dort werden als Zusatzstoffe Stilben-Derivate und ähnlich aufgebaute Verbindungen genannt.

Auch in der LU-A 50 998 sind bei Bestrahlung aufleuchtende Kosmetika beschrieben. Als fluoreszierende Zusätze sind bestimmte Pigmente genannt wie «Rotpigment HiViz» der Firma Société Lawter Chemicals und ähnliche. Auch hier ist zu bemerken, dass nur geringfügige Leuchtdichten erzielt werden können, die für Theaterzwecke meist zu schwach sind und dass insbesondere Mischfarben wegen der Eigenfarbe des Fluoreszenz-Leuchtens nicht erreichbar sind.

Bei diesem Stand der Technik besteht die Aufgabe, die bekannten, bei Bestrahlung durch ultraviolettes Licht fluoreszierenden Schminken derart weiterzuentwickeln, dass ihre Eigenfarbe durch gewollte Änderung der Lichtzusammensetzung in weiten Grenzen veränderbar ist oder dass eine spezielle Eigenfarbe hoher Leuchtdichte durch Bestrahlung mit bestimmtem Licht erst zum Vorschein gebracht wird.

Zur Lösung dieser Aufgabe wird eine feste, pastöse oder flüssige Schminke vorgeschlagen, die aus einer Schminkengrundlage besteht, einem Zusatz von 0,01 bis 2,0 Gew.% 1-Hydroxy-3,6,8-pyrentrisulfonsäure (auch 3-Hydroxypyren-5,8,10-trisulfonsäure genannt) oder einem Salz dieser Säure sowie gegebenenfalls noch weiteren in Schminken üblichen Zusätzen.

Die Verwendung von 1-Hydroxypyrentrisulfonsäure in kosmetischen Präparaten, beispielsweise Badepräparaten, ist bekannt (Chemical Abstracts Vol. 96 (1982), Ref. 168537m). Der Zusatz dieses Stoffes zu Badepräparaten erfolgt aber nicht, um diesen eine Eigenfluoreszenz zu verleihen, sondern aus anderen Gründen. Die fluoreszierenden Eigenschaften des genannten Stoffes werden sogar durch Zusatz geeigneter Säuren, beispielsweise Zitronensäure, unterdrückt.

Es hat sich gezeigt, dass Schminken der angegebenen Zusammensetzung hinsichtlich Leuchtfarbe und Leuchtdichte den bisher bekannten Präparaten deutlich überlegen sind. Wird beispielsweise eine Schminke, welche 1-Hydroxy-3,6,8-pyrentrisulfonsäure in Mengen von 0,01 bis 2,0 Gew.%, vorzugsweise 0,03 bis 0,1 Gew.% enthält, leicht sauer eingestellt, etwa auf einen pH-Wert von 3,9, so ist die Schminke bei ausschliesslicher Gegenwart von sichtbarem Licht farblos.

Wird sie mit ultraviolettem Licht von 320–400nm bestrahlt, so leuchtet sie intensiv weiss-blau.

Des weiteren wird vorgeschlagen, der Schminke ausser dem genannten fluoreszierenden Stoff auch noch andere Farbstoffe bekannter Art zuzusetzen, beispielsweise das schon erwähnte Eosin. Zwar fluoresziert Eosin bei Bestrahlung mit ultraviolettem Licht auch schwach, jedoch ist die Fluoreszenz so gering, dass sie als Eigenfarbe für Schminke nicht in Betracht kommt. Wird Eosin jedoch der zuvor erwähnten Schminke zugesetzt, so leuchtet diese bei Bestrahlung durch ultraviolettes Licht rosa. Der Effekt kommt dadurch zustande, dass die mit Eosin angefärbte Grundmasse für das vom Pyren-Derivat abgestrahlte Licht als Filter wirkt, welches in seiner Eigenfärbung, nämlich rosa, bei Bestrahlung zum Leuchten gebracht wird.

Wird eine Schminke, welche als fluoreszierenden Stoff 1-Hydroxy-3,6,8-pyrentrisulfonsäure oder ein Salz dieser Säure enhält, auf einen neutralen pH-Wert von beispielsweise 7,2 eingestellt, so erscheint die Schminke in Gegenwart von ausschliesslich sichtbarem Licht schwach-gelblich, wobei allerdings die Färbung derart gering ist, dass sie auf der Haut kaum zu erkennen ist. Bei Bestrahlung durch ultraviolettes Licht, beispielsweise mit einer Schwarzlicht-Lampe, deren Strahlung 320–400 nm beträgt, leuchtet die Schminke intensiv gelb-grün auf. Wird der Schminke zusätzlich noch ein Rotfarbstoff zugesetzt, so leuchtet die Schminke bei Bestrahlung intensiv orange.

Die vorgeschlagenen Schminken dienen einerseits Zwecken der dekorativen Kosmetik. Sie können andererseits aber auch bei Film- und Fernsehaufnahmen verwendet werden, wobei es bekanntlich erforderlich ist, die Darsteller zuvor zu schminken. Höchst unangenehm ist es, wenn die Darsteller, beispielsweise zu interviewende Politiker, vorher und nach den Aufnahmen im geschminkten Zustand bleiben müssen, bis ein fachmännisches Abschminken möglich ist. In diesem Fall können die betroffenen Personen mit den hier vorgeschlagenen, bei sichtbarem Licht weitgehend unsichtbaren Schminken geschminkt werden, wonach sie bei der eigentlichen Aufnahme zusätzlich mit Schwarzlicht, also mit einer Ultraviolett-Strahlung, die für das Auge unsichtbar bleibt, beleuchtet werden. Die Schminken leuchten dann in der gewollten Eigenfarbe auf und geben so den gewünschten Effekt. Des weiteren können die vorgeschlagenen Schminken als Theaterschminken verwendet werden.

Die Erfindung wird im folgenden anhand einiger Rezeptbeispiele erläutert.

Beispiel 1
Weiss-blau aufleuchtende, halbfeste Schminke in Stiftform:

Grundmasse:
15 Gew.% Ozokerit,
10 Gew.% Sorbitanpalmitat,
 9 Gew.% Bienenwachs,
 6 Gew.% Candelillawachs,
 5 Gew.% Carnaubawachs,
25 Gew.% Paraffinöl,
30 Gew.% Rizinusöl,

Stiftmasse:
72,95 Gew.% Grundmasse,
15,00 Gew.% Talkum,
 2,00 Gew.% Aerosil,
 4,00 Gew.% Betone,
 0,05 Gew.% 3-Hydroxypyren-5,8,10-trisulfon-
          saures Natrium,
 5,00 Gew.% Polyglykol 200,
 1,00 Gew.% Zitronensäure.

Die Schminke ist bei ausschliesslicher Anwesenheit von weissem Licht praktisch farblos. Sie leuchtet bei Bestrahlung mit ultraviolettem Licht (Wellenlänge 320–400 nm) intensiv weiss-blau.

Beispiel 2

Stiftmasse:
74,65 Gew.% Grundmasse (siehe Beispiel 1),
15,00 Gew.% Talkum,
 4,00 Gew.% Bentone,
 2,00 Gew.% Aerosil,
 0,05 Gew.% 3-Hydroxypyren-5,8,10-trisulfon-
          saures Natrium,
 0,30 Gew.% Eosin,
 3,00 Gew.% Glyzerin,
 1,00 Gew.% Zitronensäure.

Die Schminke ist bei ausschliesslicher Anwesenheit von sichtbarem Licht schwach rosa. Sie leuchtet bei Bestrahlung durch ultraviolettes Licht der Wellenlänge 350 nm intensiv rosa bis rot auf.

Beispiel 3

Stiftmasse:
84,45 Gew.% Grundmasse (siehe Beispiel 1),
 5,00 Gew.% Talkum,
 1,00 Gew.% Aerosil,
 4,00 Gew.% Bentone,
 0,05 Gew.% 3-Hydroxypyren-5,8,10-trisulfon-
          saures Natrium,
 5,00 Gew.% 1,2 Propylenglykol,
 0,50 Gew.% Triäthanolamin.

Der pH-Wert dieser Schminke beträgt 7,2. Die Schminke ist bei ausschliesslicher Anwesenheit von sichtbarem Licht sehr schwach gelblich gefärbt. Sie leuchtet bei Bestrahlung mit ultraviolettem Licht der Wellenlänge 350 nm intensiv gelbgrün auf.

Beispiel 4

Stiftmasse:
84,39 Gew.% Grundmasse,
 5,00 Gew.% Talkum,
 1,00 Gew.% Aerosil,
 4,00 Gew.% Bentone,
 0,05 Gew.% 3-Hydroxypyren-5,8,10-trisulfon-
          saures Natrium,
 0,06 Gew.% Eosin,
 5,00 Gew.% Polyglykol 400,
 0,50 Gew.% Triäthanolamin.

Der pH-Wert dieser Schminke beträgt 7,2. Die Schminke ist bei ausschliesslicher Anwesenheit von sichtbarem Licht sehr schwach orange gefärbt. Sie leuchtet bei Bestrahlung mit ultraviolettem Licht der Wellenlänge 350 nm intensiv rot.

Beispiel 5

Nagellack auf Alkoholbasis:
19,00 Gew.% Polyacrylsäureester,
 1,20 Gew.% Ammoniak, 25%ig,
24,00 Gew.% destilliertes Wasser,
 0,01 Gew.% 3-Hydroxypyren-5,8,10-trisulfon-
          saures Natrium,
ad 100 Äthylalkohol.

Der Nagellack ist bei ausschliesslicher Anwesenheit von sichtbarem Licht farblos. Er leuchtet bei Bestrahlung durch ultraviolettes Licht der Wellenlänge 350 nm intensiv weiss-grün.

Beispiel 6

Haargel:
25,00 Gew.% Polyäthylenglykol 4000,
20,00 Gew.% 1,2 Propylenglykol,
 2,00 Gew.% Polyacrylsäureester,
 1,50 Gew.% 3-Hydroxypyren-5,8,10-trisulfonsaures Natrium,
 1,00 Gew.% Zitronensäure,
ad 100 Wasser.

Das Haargel ist bei ausschliesslicher Anwesenheit von sichtbarem Licht praktisch ungefärbt. Es leuchtet bei Bestrahlung durch ultraviolettes Licht der Wellenlänge 350 nm intensiv weissgrün.

Die in den Beispielen aufgeführten Bestandteile haben die folgende Wirkung:

Bei den pastösen Stiftrezepturen dient die Ozokerit-Wachs-Mischung mit Zusätzen von Paraffinöl und Rizinusöl in bekannter Weise als Basis für die Wirkstoffe, im vorliegenden Fall insbesondere dem fluoreszierenden Farbstoff. Der Zusatz von Sorbitanpalmitat dient als Emulgator. Die Konsistenz der Grundmasse wird korrigiert durch die Zusätze Talkum, Aerosil und Bentone. Talkum dient dabei vorwiegend als Mattierungsmittel und verbessert die Gleiteigenschaften. Aerosil dient in engerem Sinne zur Konsistenzregelung, Bentone dient als Thixotropier- und Verdikkungsmittel.

Die Bestandteile Zitronensäure oder Triäthanolamin dienen zur Einstellung des pH-Wertes. Das zugefügte Polyglykol 400 oder die anderen in den Rezepturen genannten analog wirkenden Stoffe, wie 1,2 Propylenglykol, Glyzerin, Polyoxyäthylenderivate usw. dienen als Lösungsmittel für wasserlösliche Farbstoffe wie das genannte Natriumsalz der 3-Hydroxypyrentrisulfonsäure.

Die sonst noch vorhandenen Farbstoffe dienen der Einstellung spezieller Eigenfarben; das Natriumsalz der 3-Hydroxypyrentrisulfonsäure dient als fluoreszierender Stoff.

## Patentanspruch

Feste, pastöse oder flüssige Schminke, bestehend aus Schminken-Grundlage, einem Zusatz von 0,01 bis 2,0 Gew.% 1-Hydroxy-3,6,8-pyrentrisulfonsäure oder einem Salz dieser Säure sowie gegebenenfalls noch weiteren, in Schminken üblichen Zusätzen.

## Revendication

Maquillage solide, pâteux ou liquide, consistant en une base de maquillage, une addition de 0,01 à 2,0% en poids d'acide 1-hydroxy-3,6,8-pyrènetrisulfonique ou de sels de cet acide ainsi éventuellement que d'autres additifs habituels dans les maquillages.

## Claim

Solid, pasty or liquid make-up comprising make-up base, an additive of 1 hydroxy-3,6,8 pyrene trisulfonic acid in amounts from 0,01 to 2,0% by weight or a salt thereof and alternatively further additives usual in make-up.